# EUROPEAN PATENT APPLICATION

(11) **EP 4 534 989 A1**
(43) Date of publication of application: **09.04.2025**
(21) Application number: 23810665.2
(22) Date of filing: 31.03.2023
(51) Int. Cl.: G01N 27/12, A61F 13/42

(54) **METABOLITE DETECTION APPARATUS, DIAPER AND NURSING SYSTEM**

(30) Priority: 27.05.2022 CN 202210589195
(71) Applicant: Shenda Chuangxin (Shenzhen) Technology Co., Ltd, Shenzhen, Guangdong 518051 (CN)
(72) Inventor: XIE, Jiquan, Shenzhen, Guangdong 518051 (CN); XIE, Junda, Shenzhen, Guangdong 518051 (CN)
(74) Representative: Noble, Nicholas
(86) International application number: PCT/CN2023/085439
(87) International publication number: WO 2023/226590

(57) **Abstract**

A metabolite detection apparatus, a diaper and a nursing system. The metabolite detection apparatus is applied to a diaper, and comprises: a sensing module (100), which comprises a sensing unit (110), a signal conditioning chip (120) and a first coil (130), which are sequentially connected, wherein the sensing unit (110) is used for sensing a metabolite and generating a plurality of corresponding sensing signals, and the signal conditioning chip (120) is used for processing the sensing signals to respectively generate corresponding conditioning signals, and transmitting a plurality of conditioning signals to the first coil (130) for sending; and a processing module (200), which comprises a second coil (210) and a data processing unit (220), which are connected to each other, wherein the second coil (210) is electromagnetically coupled and connected to the first coil (130) to wirelessly transmit energy and data, the second coil (210) is used for receiving the plurality of conditioning signals and transmitting the plurality of conditioning signals to the data processing unit (220), and the data processing unit (220) is used for acquiring metabolite information according to the plurality of conditioning signals.

## Description

### TECHNICAL FIELD

The present disclosure relates to the field of diaper detection technology, and particularly to a metabolite detection apparatus, a diaper and a care system.

### BACKGROUND

Diapers, disposable diapers, sanitary napkins, nursing pads, etc., are widely used by infants, women, and people with incontinence. The diapers are made of flexible substrates such as PI, PET, nylon, and non-woven fabrics. The absorption portion is usually in the shape of a long strip, which is configured to absorb or adhere to metabolites when worn to prevent leakage of the metabolites.

In order to identify types of the metabolites, multiple kinds of sensors are usually provided in the diapers. However, connection of each sensor to a processor for data processing and analysis may result in excessive wiring, which increases the complexity of the lines and makes quality control more difficult.

### SUMMARY

In view of this, it is necessary to provide a metabolite detection apparatus with a simple line, a diaper, and a nursing system.

A metabolite detection apparatus is provided, applied to a diaper, the metabolite detection apparatus includes:
a sensor module, including a sensor unit, a signal conditioner chip, and a first coil connected to each other in sequence, in which the sensor unit is configured to sense a metabolite and generate a plurality of corresponding sensing signals, the signal conditioner chip is configured to process each of the sensing signals to generate a plurality of corresponding conditioning signals respectively, and transmit the plurality of conditioning signals to the first coil for transmissions;
a processing module, including a second coil and a data processing unit connected to each other, in which the second coil is electromagnetically coupled to the first coil to wirelessly transmit energy and data, the second coil is configured to receive the plurality of conditioning signals and transmit the plurality of conditioning signals to the data processing unit, and the data processing unit is configured to acquire metabolite information according to the plurality of conditioning signals.

In an embodiment, the sensor unit includes a plurality of sensors, each of which is provided on a diffusion path of the metabolite, and each of the sensors is configured to generate a corresponding sensing signal;
the signal conditioner chip is configured with a plurality of sensing channel interfaces, each of which is configured to connect to a corresponding sensor.

In an embodiment, the signal conditioner chip is configured with an updateable signal conditioning algorithm, and the signal conditioning algorithm is set to correspond to types of the plurality of sensors in the sensor unit.

In an embodiment, the signal conditioner chip is provided with preset identity information,
when the signal conditioner chip transmits the plurality of conditioning signals to the first coil for transmissions, the signal conditioner chip is further configured to transmit the identity information to the first coil for the transmission, to allow the processing unit to establish a mapping relationship between the metabolite information and the identity information.

In an embodiment, the data processing unit is further configured to generate and transmit a data acquisition instruction via the second coil,

the signal conditioner chip is further configured to receive the data acquisition instruction via the first coil, and transmit the conditioning signal back to the data processing unit in response to the data acquisition instruction.

In an embodiment, the sensor unit includes:
a plurality of humidity sensors, respectively provided at different sensing points on the diffusion path of the metabolite, and each humidity sensor being configured to sense a humidity at a sensing point and generate a corresponding humidity sensing signal;
the data processing unit is further configured to determine a metabolite type according to the humidity sensed by each humidity sensor, and the metabolite type includes a liquid metabolite and a non-liquid metabolite.

In an embodiment, the sensor unit includes:
at least one temperature sensor, provided on the diffusion path of the metabolite, in which the temperature sensor is configured to sense a temperature at a sensing point and generate a corresponding temperature sensing signal;
the data processing unit is further configured to determine a metabolite type according to the temperature sensed by the temperature sensor, the metabolite type includes a liquid metabolite and a non-liquid metabolite.

In an embodiment, the sensor unit includes:
a liquid-phase metabolite sensor, provided in a biochemical reaction region of the diaper, and configured to sense a liquid flowing into the biochemical reaction region from a liquid siphon channel of the diaper and generate a corresponding liquid-phase biochemical sensing signal;
the data processing unit is further configured to acquire liquid-phase biochemical information corresponding to the liquid-phase biochemical sensing signal.

In an embodiment, the sensor unit includes a gas-phase metabolite sensor and a gas latching structure covering the gas-phase metabolite sensor, the gas latching structure is configured to latch the gas in the metabolite, and the gas-phase metabolite sensor is configured to sense the latched gas and generate a corresponding gas-phase biochemical sensing signal;
the data processing unit is further configured to acquire gas-phase biochemical information corresponding to the gas-phase biochemical sensing signal.

In an embodiment, the processing module further includes at least one of an operation unit, an indicator unit, or a power management unit,
the operation unit is connected to the data processing unit, and is configured to receive an activation operation input by a user and generate an activation signal accordingly, and transmit the activation signal to the data processing unit;
the indicator unit is connected to the data processing unit, and is configured to receive an indication signal output by the data processing unit, and indicate the user according to status information carried by the indication signal, the status information carried by the indication signal includes at least one of status information of the metabolite, status information of the sensor module, or status information of the processing unit; and
the power management unit is connected to the data processing unit and is configured to supply power to the data processing unit.

A diaper is provided, including:
a surface layer;
a bottom layer;
at least one inner core layer provided between the surface layer and the bottom layer;
a sensor layer, provided between the surface layer and the bottom layer, in which at least one inner core layer is provided between the sensor layer and the bottom layer;
the above-mentioned metabolite detection apparatus, the sensor module in the metabolite detection apparatus is provided in the sensor layer, and the processing module in the metabolite detection apparatus is provided on an outer side of the bottom layer.

In an embodiment, there is no inner core layer provided on a coupling path between the first coil and the second coil in the metabolite processing apparatus.

In an embodiment, a partial inner core layer is provided on a coupling path between the first coil and the second coil in the metabolite processing apparatus;
the processing unit is further configured to acquire an electromagnetic coupling efficiency on the coupling path, and acquire the amount of metabolite absorbed by the inner core layer according to the electromagnetic coupling efficiency.

In an embodiment, the diaper according further includes:
a receiving bag connected to an outer side of the bottom layer and configured to receive the processing module, the receiving bag is provided with an opening, a switch structure is provided at the opening, the switch structure is configured to expose the opening when opened, to put in or take out the processing module through the opening, and the switch structure is further configured to close the opening when closed, to prevent the processing module from falling off during an operation.

In an embodiment, the receiving bag is provided at a position corresponding to the first coil in the sensor module to minimize a coupling path between the second coil and the first coil.

A nursing system is provided, including:
a mobile terminal including a first wireless transmission unit;
the above-mentioned diaper, the processing module of the diaper further includes a second wireless transmission unit which is connected to a data processing unit and is configured to transmit metabolite information to the first wireless transmission unit of the mobile terminal, and receive a control instruction from the first wireless transmission unit.

In an embodiment, the nursing system further includes a cloud server, the mobile terminal is further configured to transmit the metabolite information to the cloud server, and the cloud server is configured to store and analyze the received metabolite information.

The metabolite detection apparatus in the above embodiment includes a sensor module and a processing module. The sensor module includes a sensor unit, a signal conditioner chip, and a first coil connected to each other in sequence. The processing module includes a second coil and a data processing unit connected to each other. By providing the signal conditioner chip and establishing the wireless communication between the sensor unit and the data processing unit via the first coil and the second coil, the connection length of the line is effectively reduced and the complexity of the line is simplified. Furthermore, since the first coil and the second coil can transmit energy by the wireless coupling mode, in the sensor module, the energy wirelessly received by the first coil can be directly utilized to power the sensor unit, the signal conditioner chip and other devices. Based on the above power supply mode, there is no need to provide a battery and other structures in the sensor module, thereby simplifying the structure of the sensor module and further simplifying the overall structure of the diaper.

### BRIEF DESCRIPTION OF THE DRAWINGS

In order to more clearly illustrate the technical solution in the embodiments of the present disclosure or the conventional technology, accompanying drawings required for description of the embodiments or the conventional technology are briefly introduced below. Obviously, the drawings described below are merely some embodiments of the present disclosure, and thoses skilled in the art can obtain other drawings based on these drawings without any creative work.
FIG. 1 is a schematic diagram of a system structure of a metabolite detection apparatus according to an embodiment.
FIG. 2 is a schematic diagram I of a sensor according to an embodiment.
FIG. 3 is a schematic diagram of a humidity variation curve of a semi-solid metabolite according to an embodiment.
FIG. 4 is a schematic diagram of a humidity variation curve of a liquid metabolite according to an embodiment.
FIG. 5 is a schematic diagram II of a sensor according to an embodiment.
FIG. 6 is a schematic position diagram of a humidity sensor according to an embodiment.
FIG. 7 is a schematic diagram III of a sensor according to an embodiment.
FIG. 8 is a cross-sectional view of a liquid-phase metabolite sensor according to an embodiment.
FIG. 9 is a schematic structure diagram of a processing module according to an embodiment.
FIG. 10 is a cross-sectional view I of a diaper according to an embodiment.
FIG. 11 is a cross-sectional view II of a diaper according to an embodiment.
FIG. 12 is a schematic structure diagram of a nursing system according to an embodiment.

### Reference signs:

100, sensor module; 110, sensor unit; 120, signal conditioner chip; 130, first coil; 200, processing module; 210, second coil; 220, data processing unit; 230, operation unit; 240, indicator unit; 250, power management unit; 260, second wireless transmission unit; 11, surface layer; 12, bottom layer; 13, inner core layer; 14, sensor layer.

### DETAILED DESCRIPTION

In order to facilitate the understanding of the present disclosure, the present disclosure will be described more comprehensively below with reference to the relevant accompanying drawings. Embodiments of the present disclosure are shown in the accompanying drawings. However, the present disclosure may be implemented in many different forms and is not limited to the embodiments described herein. Rather, these embodiments are provided to make the present disclosure more thorough and comprehensive.

Unless defined otherwise, all technical and scientific terms used herein have the same meaning as commonly understood by one of ordinary skill in the art to which the present disclosure belongs. The terms used herein in the specification of the present disclosure are only for the purpose of describing specific embodiments and are not intended to limit the present disclosure.

It should be appreciated that the terms "first", "second", etc., used in the present disclosure may be used for describing various elements, but these elements are not limited by these terms. These terms are only used for distinguishing a first element from another element. For example, a first resistor could be referred to as a second resistor, and, similarly, a second resistor could be referred to as a first resistor without departing from the scope of the present disclosure. The first resistor and the second resistor are both resistors, but they are not the same resistor.

It should be appreciated that the "connection" in the following embodiments should be understood as "electrical connection", "communication connection", etc., when the connected circuits, modules, units, etc., have electrical signals or data transmission with each other.

As used herein, the singular forms "a", "an" and "the" may include the plural forms as well, unless the context clearly indicates otherwise. It should also be appreciated that the terms "including/comprising" or "having" and the like specify the presence of the stated features, wholes, steps, operations, components, portions or combinations thereof, but do not exclude the possibility of the presence or addition of one or more other features, wholes, steps, operations, components, portions or combinations thereof. Meanwhile, the term "and/or" used in this specification includes any and all combinations of the relevant listed items.

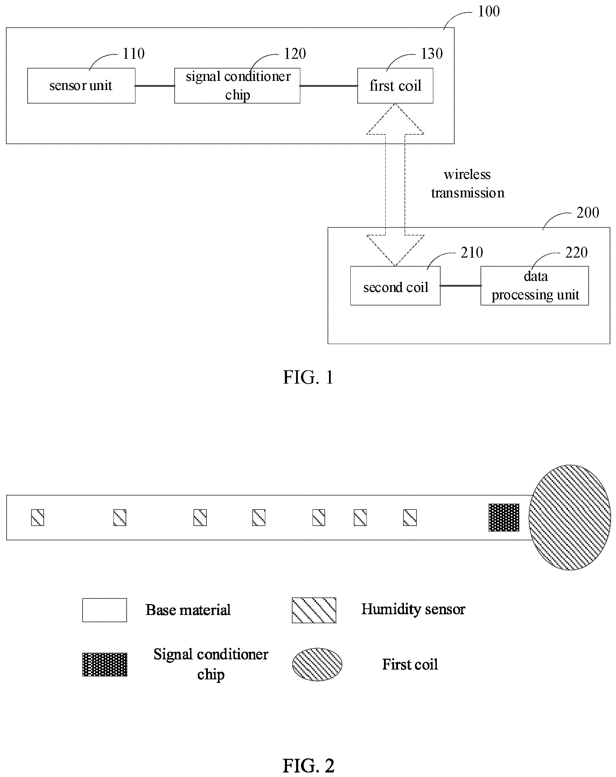

FIG. 1 is a schematic diagram of a system structure of a metabolite detection apparatus according to an embodiment. The metabolite detection apparatus according to the embodiment is applied to a diaper. Referring to FIG. 1, in the embodiment, the metabolite detection apparatus may include a sensor module 100 and a processing module 200.

The sensor module 100 may include a sensor unit 110, a signal conditioner chip 120, and a first coil 130 which are connected in sequence. The sensor unit 110 is configured to sense a metabolite and generate a plurality of corresponding sensing signals. The sensor unit 110 may include a plurality of sensors, each of which is provided on a diffusion path of a metabolite, and each of the sensors is configured to generate a corresponding sensing signal. The signal conditioner chip 120 is configured to process each of the sensing signals to generate a corresponding conditioning signal respectively, and transmit a plurality of conditioning signals to the first coil 130 for transmissions. Specifically, the signal conditioner chip 120 may have a signal modulation function, for example, the signal conditioner chip 120 can amplify and enhance the relatively weak signal outputted by the sensor unit 110; or the signal conditioner chip 120 can also regulate a voltage of a signal when a voltage range of the signal does not meet a voltage input requirement of a subsequent module; or the signal conditioner chip 120 can also filter out noises other than the signal, thereby outputting a cleaner conditioning signal. The signal conditioner chip 120 may also have at least one of the functions of a digital-to-analog conversion, a non-transitory storage, a simple logic operation, and a wireless modulation, etc.

The processing module 200 may include a second coil 210 and a data processing unit 220 connected to each other. The second coil 210 is electromagnetically coupled to the first coil 130 to wirelessly transmit energy and data. The second coil 210 is configured to receive a plurality of conditioning signals and transmit the plurality of conditioning signals to the data processing unit 220. It should be appreciated that during the use, the signal conditioner chip 120 may transmit data to the processing module 200 at regular intervals; alternatively, the signal conditioner chip 120 may transmit data back in response to an acquisition instruction of the processing module 200, which is not limited in the embodiment. The data processing unit 220 is configured to acquire metabolite information according to the plurality of conditioning signals. Specifically, the metabolite information may include, but is not limited to, the metabolite type. After receiving the conditioning signal, the processing module 200 may determine a sensing result of a sensing point according to the conditioning signal, and further determine the metabolite type according to the sensing result.

In the embodiment, compared to the related technology in which the sensor unit 110 is directly connected to the processing module 200 via a line, the signal conditioner chip 120 is provided therebetween, and the signal conditioner chip controls the first coil 130 to send and receive signals. The wired connection between the sensor unit 110 and the processing module 200 can be converted into a wireless connection, thereby reducing a connection length of the line and simplifying the complexity of the line. In addition, since the line connection between the processing module 200 and the signal conditioner chip 120 is omitted, the processing module 200 does not require a connector for the line connection. Accordingly, a rigid reinforcement plate can be removed, the reinforcement process is omitted, so that the rolling process is easier to perform, thereby saving materials and process costs, and improving the processing efficiency. Meanwhile, the convenience for users during use and assembly is greatly improved, and the function of the sensor module is easily extended. Furthermore, since the first coil and the second coil can transmit energy by wireless coupling, in the sensor module, the energy wirelessly received by the first coil can be directly utilized to power the sensor unit, the signal conditioner chip and other devices. Based on the above power supply method, there is no need to set up batteries and other structures in the sensor module, thereby simplifying the structure of the sensor module and further simplifying the overall structure of the diaper.

In an embodiment, when the sensor unit 110 includes a plurality of sensors, the signal conditioner chip 120 is configured with a plurality of sensing channel interfaces, each of which is configured to connect to one corresponding sensor. It should be appreciated that the sensing channel interface can be designed according to the requirements, so it is easier to adapt to the situation where the number of sensors increases. Furthermore, since the signal conditioner chip 120 has a smaller size, the added sensing channel interface may not have a significant impact on the overall space occupied by the device. It should be appreciated that when the number of sensors increases to sense more pieces of data, the compatibility can be implemented by simply changing the connection relationship between the sensor and the sensing channel interface. The method is simple and easy to be implemented, and is more conducive to extending the function of the metabolite detection device.

In an embodiment, the signal conditioner chip 120 is configured with an updateable signal conditioning algorithm, and the signal conditioning algorithm is set corresponding to the types of the multiple sensors in the sensor unit 110. That is, more abundant signal conditioning functions can be implemented only by updating the signal conditioning algorithm in the signal conditioner chip 120. Accordingly, there is no need to update the hardware structure of the signal conditioner chip 120, and only a simple software algorithm update is required to complete the update and iteration of the product.

In an embodiment, the signal conditioner chip 120 is further configured with preset identity information. Accordingly, the signal conditioner chip 120 has a storage function to record identity information of each diaper. The identity information includes but is not limited to ID information of the diaper and other information. When the signal conditioner chip 120 transmits the plurality of conditioning signals to the first coil 130 for transmissions, the signal conditioner chip 120 is further configured to transmit the identity information to the first coil 130 for the transmission, so that the processing unit establishes a mapping relationship between the metabolite information and the identity information. In the embodiment, the processing module 200 may determine the corresponding relationship between the metabolite information and the signal conditioner chip 120. Furthermore, when a corresponding relationship between the identity information of the signal conditioner chip 120 and user usage information is established, a user name, diaper wearing time, etc., corresponding to each piece of metabolite information can also be acquired, thereby facilitating traceability and data analysis.

In an embodiment, the data processing unit 220 is further configured to generate and transmit a data acquisition instruction via the second coil 210. The signal conditioner chip 120 is further configured to receive the data acquisition instruction via the first coil 130, and transmit the conditioning signal back to the data processing unit 220 in response to the data acquisition instruction. It should be appreciated that the user has a need to open the APP on the mobile terminal and query the current usage of the diaper to determine whether the diaper needs to be replaced. Accordingly, by setting the above functions, the data can be acquired in real time, thereby providing accurate information to the user and improving the user experience.

FIG. 2 is a schematic diagram of a sensor according to an embodiment. Referring to FIG. 2, in an embodiment, the sensor unit 110 includes a plurality of humidity sensors, and the plurality of humidity sensors are all provided on a substrate of the sensor layer 14. The substrate is a flexible material to fit the user's body, and a conductive circuit is embedded in the substrate to transmit the sensing signal of each sensor. The plurality of humidity sensors are respectively provided at different sensing points on the diffusion path of the metabolite, and each humidity sensor is configured to sense the humidity at the sensing point and generate a corresponding humidity sensing signal. The number of humidity sensors may be 5 to 10, which can be determined according to the size of the diaper. The data processing unit 220 is further configured to determine the metabolite type according to the humidity sensed by each humidity sensor, wherein the metabolite type includes a liquid metabolite and a non-liquid metabolite. Specifically, the processing module 200 may acquire growth status information according to sensing results of the sensors, and determine the metabolite type according to the growth status information. The growth status information is configured to characterize a growth situation indicated by the sensing result of each sensing point over time, for example, the growth status information may include a humidity growth rate, maintenance time of the humidity within a preset humidity range after the humidity increases, a situation of maintaining the temperature within a preset temperature range after the humidity increases, etc.

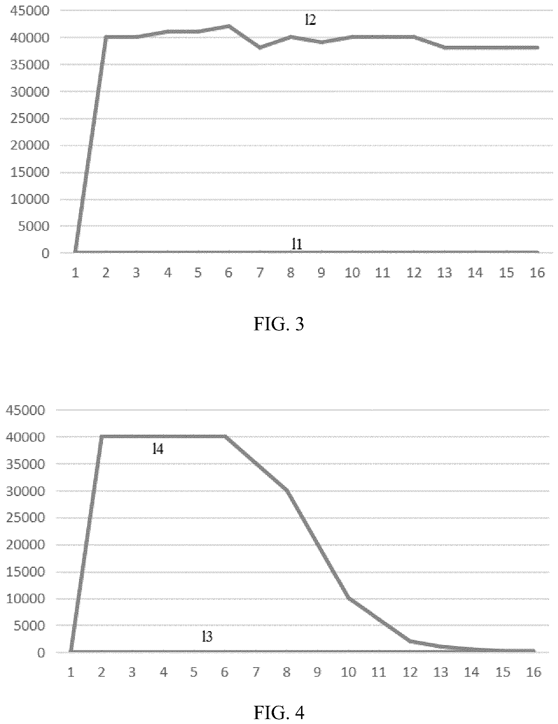

As an example, the processing module 200 is further configured to determine that the metabolite type is the liquid metabolite when the humidity growth rate of at least one sensing point is greater than a rate threshold. The rate threshold can be determined according to an empirical value of the humidity growth rate when the liquid metabolite diffuses in an inner core layer of the diaper. In another example, the processing module 200 may also be configured to: determine that the metabolite type is a semi-solid metabolite when the maintenance time of at least one sensing point is greater than a first time threshold; and/or determine that the metabolite type is a liquid metabolite when the maintenance time of any sensing point is less than a second time threshold. The semi-solid metabolite refers to a metabolite with liquid stools, for example, loose stools. It should be appreciated that the first time threshold can be determined according to a usual duration of urination of the user, for example, the first time threshold may be greater than or equal to the maximum duration of urination of the user. Since the liquid metabolite is quickly absorbed by the inner core layer of the diaper after being discharged, the moisture can only be maintained under the condition of continuous urination. Since the semi-solid metabolite carries a liquid such as the liquid in the metabolite, and the liquid in the metabolite may not be quickly and completely absorbed by the inner core layer of the diaper, the humidity at the sensing point can be maintained for a period of time. If the maintenance time of at least one sensing point exceeds the first time threshold, that is, exceeds the normal duration of urination of the user, it indicates that the maintenance of the humidity at the sensing point is not caused by the urination, but by the semi-solid metabolite. Accordingly, the metabolite type can be determined as the semi-solid metabolite. FIG. 3 is a schematic diagram of a humidity variation curve of a semi-solid metabolite according to an embodiment. Referring to FIG. 3, the curve 11 is a time variation curve, and the curve 12 is a humidity value variation curve of the semi-solid metabolite. The liquid metabolite may be, for example, liquid, blood in the metabolite, etc. The second time threshold may also be determined according to the usual duration of urination of the user, for example, may be less than or equal to the maximum duration of urination. It should be appreciated that if the maintenance time of at least one sensing point is less than the first time threshold, that is, less than the normal duration of urination of the user, it can be indicated that the maintenance of the humidity at the sensing point is caused by the urination, so that this moment the metabolite type can be determined as the liquid metabolite. FIG. 4 is a schematic diagram of a humidity variation curve of a liquid metabolite according to an embodiment. Referring to FIG. 4, the curve 13 is a time variation curve, and curve 14 is a humidity value variation curve of the liquid metabolite. The first time threshold may be equal to the second time threshold.

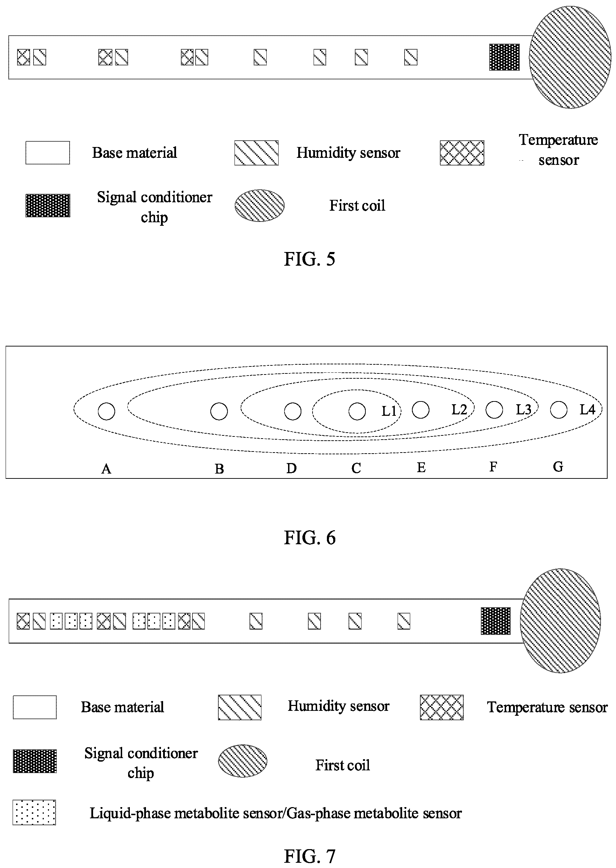

FIG. 5 is a schematic diagram II of a sensor according to an embodiment. Referring to FIG. 5, in an embodiment, the sensor unit 110 includes at least one temperature sensor, which is provided on a diffusion path of a metabolite. The temperature sensor is configured to sense a temperature at a sensing point and generate a corresponding temperature sensing signal. In an embodiment shown in FIG. 5, the sensor unit 110 includes three temperature sensors. The data processing unit 220 is further configured to determine the metabolite type according to the temperature sensed by the temperature sensor, the metabolite type includes a liquid metabolite and a non-liquid metabolite.

Specifically, the growth status information may further include maintenance time of a temperature within a preset temperature range after the humidity increases. The processing module 200 is further configured to determine the metabolite type as a semi-solid metabolite when the growth status information of at least one humidity sensing point satisfies a preset condition while a temperature of an adjacent temperature sensing point gradually decreases. It should be appreciated that when the metabolite is the semi-solid metabolite, since the liquid in the metabolite are not be quickly and completely absorbed by the inner core layer of the diaper, the humidity thereof may be maintained within a certain range after the metabolite is excreted. In addition, the temperature of the metabolite may slowly decrease after the metabolite is excreted, rather than continuing to increase or being maintained in a certain temperature range like in the case of the continuous urination. Therefore, for a certain sensing point, when the humidity of the sensing point increases and then is maintained in the preset humidity range, and the temperature of the sensing point gradually decreases, the metabolite type can be determined as the semi-solid metabolite.

In an embodiment, the data processing unit 220 is further configured to determine a target number according to the humidity, in which the target number is the number of sensing points at which the humidity is greater than the second humidity threshold, and determine a metabolite amount according to the target number. The second humidity threshold may be a humidity of the inner core layer of the diaper when the liquid metabolite is excreted into the inner core layer of the diaper. It should be appreciated that if the humidity at the sensing point is greater than the second humidity threshold, it means that there exists the liquid metabolite at the sensing point. By reasonably setting a distance between the sensing points, the number of sensing points in contact with the liquid metabolite corresponds to the metabolite amount in a one-to-one correspondence, so that the corresponding metabolite amount can be obtained according to the number of sensing points.

FIG. 6 is a schematic diagram illustrating positions of humidity sensors according to an embodiment. Referring to FIG. 6, the humidity sensors are respectively arranged at humidity sensing points A, B, C, D, E, F, and G in a one-to-one correspondence, and penetration regions L1 to L4 can be divided based on the sensing points, in addition areas of the penetration regions L1 to L4 increases in sequence. When only one sensing point is in contact with the liquid excrement, the liquid excrement is located in the region L1, and the metabolite amount corresponding to the region L1 may be equal to 20 ml. When three sensing points are in contact with the liquid excrement, the liquid excrement is located in the region L2, and the metabolic amount corresponding to the region L2 may be equal to 40 ml. By analogy, the metabolic amount corresponding to the region L2 may be equal to 40 ml, and the metabolic amount corresponding to the region L4 may be equal to 120 ml. Accordingly, in the embodiment, the corresponding metabolic amount can be determined according to the target number. The method is simple and can effectively measure the metabolic amount.

In some embodiments, in order to determine the metabolic amount, the metabolic amount can also be further determined by acquiring an excretion duration of the liquid metabolite in a user's excretion process in combination with an excretion volume per unit time. The excretion duration refers to a time interval between the moment when the user starts an excretion and the moment when the user ends the excretion. The moment when the user starts the excretion can be represented by a moment when the humidity at a user urine excretion sensing point increases. Since the liquid excrement is quickly absorbed after being discharged, if the excretion stops, the humidity at the sensing point may decrease. Therefore, the moment when the user ends the excretion can be represented by a decay moment of the humidity at the user urine excretion sensing point. The humidity at the sensing point increases sharply at a growth moment, so that the growth moment can be determined according to whether the growth rate of the humidity at the target sensing point reaches a growth threshold. The humidity at the sensing point may decay sharply at the decay moment, so that the decay moment can be determined according to whether the decay rate reaches a decay threshold. The collected humidity may have a time stamp, so that the growth moment and the decay moment can be determined according to the humidity with the time stamp.

In an embodiment, the temperature sensor is further configured to at least sense a temperature at a temperature sensing point of the user stool excretion, and transmits the temperature to the processing module 200 through the signal conditioner chip 120. At least one sensor is provided at the temperature sensing point to sense the humidity at the temperature sensing point. The processing module 200 is further configured to determine the metabolite type as the solid metabolite when the temperature at the temperature sensing point rises and the humidity at an adjacent humidity sensing point is less than a first humidity threshold. It should be appreciated that the first humidity threshold may be less than or equal to the maximum humidity of the inner core layer of the diaper when the solid metabolite is excreted into the inner core layer of the diaper. When the temperature at the first sensing point rises, it indicates that there exists a metabolite at the first sensing point. When the humidity at the first sensing point is less than the first humidity threshold, it indicates that the metabolite is a solid metabolite, for example, the solid metabolite may be a dry stool.

In an embodiment, the temperature sensor is further configured to sense the temperature at a plurality of sensing points on the inner core layer of the diaper corresponding to each excretion of the user when the diaper is worn. The data processing unit 220 is further configured to determine temperature diffusion information according to the temperature, and determine the metabolite type according to the temperature diffusion information. The temperature diffusion information is configured to indicate an order in which the temperature at the sensing points rises. Specifically, when the temperature diffusion information indicates that the temperature changes successively from the sensing point at the urine to the sensing point at the stool, it is determined that the metabolite type is the liquid metabolite. When the temperature diffusion information indicates that the temperature changes successively from the sensing point at the stool to the sensing point at the urine, it is determined that the metabolite type is a non-liquid metabolite. It should be appreciated that the specific metabolite type can be determined according to the excretion position and diffusion condition of the metabolite. The liquid metabolite may include a liquid or a blood in the metabolite. The non-liquid metabolite may include a solid metabolite, such as a dry stool, and may further include a semi-solid metabolite, such as a loose stool.

FIG. 7 is a schematic diagram III of a sensor according to an embodiment. Referring to FIG. 7, in an embodiment, the sensor unit 110 may further include a liquid-phase metabolite sensor. The liquid-phase metabolite sensor is provided in a biochemical reaction region of the diaper, and is configured to sense a liquid flowing into the biochemical reaction region from a liquid siphon channel of the diaper, and generate a corresponding liquid-phase biochemical sensing signal. The data processing unit 220 is further configured to acquire liquid-phase biochemical information corresponding to the liquid-phase biochemical sensing signal.

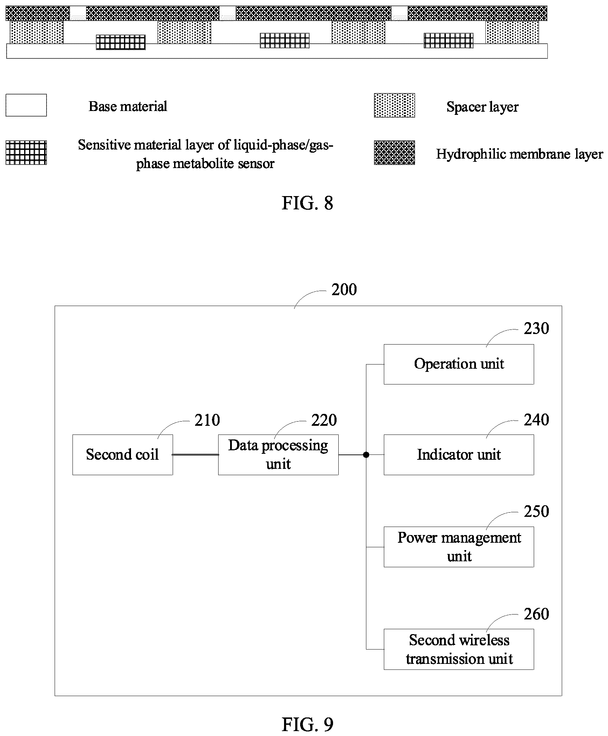

Specifically, the liquid-phase metabolite sensor is designed with a separate liquid siphon channel and a biochemical reaction region to ensure the detection of multiple kinds of metabolites, so that the detection results are not affected by a droplet flow channel and signals therebetween. The substances that can be detected include, but are not limited to, a pH value, glucose, dopamine, uric acid, ascorbic acid, galactose, ketone bodies, leukocytes, protein, occult blood, K+ ions, Na+ ions, iodine ions, calcium ions, iron ions, zinc ions, nitrites, etc. Specifically, FIG. 8 is a cross-sectional view of a liquid-phase metabolite sensor according to an embodiment. Referring to FIG. 8, a sensitive material layer of the liquid-phase metabolite sensor is provided at each sensing point on the substrate and configured to generate an electrical signal when metabolic substances undergo specific reactions. A spacer layer is provided between two adjacent sensitive material layers, which is configured to space the adjacent sensitive material layers and construct a siphon channel to latch the liquid in the metabolite. A hydrophilic membrane layer is provided on the spacer layer, and is configured to absorb the liquid in the metabolite, to guide the liquid into the siphon channel. In the embodiment shown in FIG. 8, an inlet of the siphon channel for liquid-phase component analysis is implemented by drilling holes in the hydrophilic membrane layer. It should be appreciated that liquid-phase component analysis requires the establishment of a stable reaction region to ensure that the liquid in the metabolite in the reaction region is sufficient within the detection duration. Otherwise, the liquid in the metabolite may be absorbed by the inner core layer of the diaper and cannot satisfy the requirements of the biochemical detection. The above structure provides a stable reaction region. The liquid in the metabolite enters the reaction region through siphoning and is detected by the sensitive material layer in the reaction region, thereby implementing the liquid-phase analysis.

Referring to FIG. 7, in an embodiment, the sensor unit 110 includes a gas-phase metabolite sensor and a gas latching structure covering the gas-phase metabolite sensor. The gas latching structure is configured to latch the gas in the metabolite, and the gas-phase metabolite sensor is configured to sense the latched gas and generate a corresponding gas-phase biochemical sensing signal. The data processing unit 220 is further configured to acquire gas-phase biochemical information corresponding to the gas-phase biochemical sensing signal. Similar to the siphon channel, the gas latching structure can establish a stable reaction region, thereby ensuring that the gas in the reaction region is relatively stable in the detection duration. Otherwise, the gas in the metabolite may evaporate and cannot satisfy the requirements of the biochemical detection. The above structure provides a stable reaction region, and the gas in the metabolite is fixed in the reaction region by the gas latching structure and detected by the sensitive material layer in the reaction region, thereby implementing the gas-phase analysis. The gas-phase analysis substances include (but are not limited to) ammonia, hydrogen sulfide, hydrogen, skatole, etc.

FIG. 9 is a schematic structure diagram of a processing module 200 according to an embodiment. Referring to FIG. 9, in an embodiment, the processing module 200 may further include at least one of an operation unit 230, an indicator unit 240, and a power management unit 250.

Specifically, the operation unit 230 is connected to the data processing unit 220, and is configured to receive an activation operation input by a user and generate an activation signal accordingly, and transmit the activation signal to the data processing unit 220. The activation signal can be that when the processing module 200 is placed in a receiving bag, the processing module 200 can automatically detect the sensor module 100 and transmit an activation signal to the sensor module 100 to establish communication with the sensor module 100, thereby acquiring ID information, production time, configuration information, and information indicating whether the sensor module is used, and other information of the sensor module 100. The indicator unit 240 is connected to the data processing unit 220, and is configured to receive an indication signal output by the data processing unit 220, and indicate the user according to status information carried by the indication signal. The status information carried by the indication signal may include at least one of status information of the metabolite, status information of the sensor module 100, and status information of the processing unit. The status information of the metabolite refers to the number of excretions, a duration of use, whether a detection index exceeds a set value, and so on. The status information of the sensor module 100 refers to a switch status of the sensor, an electromagnetic coupling status of the coil, and so on. The status information of the processing unit refers to whether the processing module 200 operates normally, whether the power of the processing module 200 is normal, etc. It should be appreciated that in order to prevent excessive accumulation of the liquid metabolite in the diaper from affecting the subsequent absorption of the diaper and even affecting the health of the user, the indicator unit 240 may output a warning message to remind the user to change the diaper when the metabolic amount of liquid metabolites exceeds a set warning value. The warning information may be a sound signal. The power management unit 250 is connected to the data processing unit 220 and is configured to supply power to the data processing unit 220.

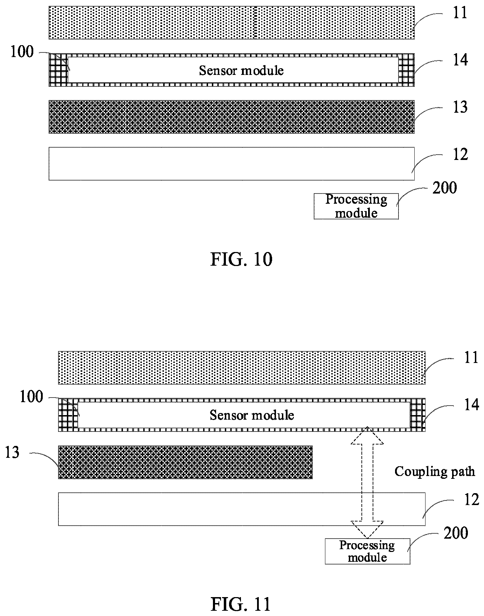

In an embodiment of the present disclosure, a diaper is further provided. FIG. 10 is a cross-sectional view of a diaper according to an embodiment. Referring to FIG. 10, the diaper includes a surface layer 11, a bottom layer 12, at least one inner core layer 13, and a sensor layer 14. One inner core layer 13 is shown in the figure. The surface layer 11 approaches the user's skin, and the bottom layer 12 is away from the user's skin. The inner core layer 13 is provided between the surface layer 11 and the bottom layer 12, such as the above-mentioned metabolite detection apparatus. The inner core layer 13 may also be referred to as an absorption layer for absorbing the metabolite. The sensor layer 14 is provided between the surface layer 11 and the bottom layer 12, and at least one inner core layer 13 is provided between the sensor layer 14 and the bottom layer 12. For example, when the diaper includes three inner core layers 13 stacked in sequence, the sensor layer 14 can be provided between the first inner core layer 13 and the surface layer 11, or between the first inner core layer 13 and the second inner core layer 13, or between the second inner core layer 13 and the third inner core layer 13, or between the third inner core layer 13 and the bottom layer 12, which is not limited in the embodiment. The sensor module 100 in the metabolite detection apparatus is provided in the sensor layer 14, that is, the sensor unit 110, the signal conditioner chip 120 and the first coil 130 in the metabolite detection apparatus are all provided on the sensor layer 14. Moreover, the processing module 200 in the metabolite detection apparatus is provided on the outer side of the bottom layer 12, that is, the second coil 210 and the data processing unit 220 in the metabolite detection apparatus are both provided on the outer side of the bottom layer 12. In the embodiment, the processing module 200 is provided on a side away from the user's skin, so that the processing module 200 can be replaced at any moment, and the metabolite can also be prevented from flowing into the processing module 200 to affect the operation of the processing module 200.

Referring to FIG. 10, in an embodiment, a partial inner core layer 13 is provided on the coupling path between the first coil 130 and the second coil 210 in the metabolite processing apparatus. The processing unit is further configured to acquire an electromagnetic coupling efficiency on the coupling path, and acquire the amount of metabolite absorbed by the inner core layer 13 according to the electromagnetic coupling efficiency. It should be appreciated that a moisture absorption status of the inner core layer 13 may affect the electromagnetic coupling efficiency. Therefore, the designed algorithm can also be used for determining the amount of metabolites and whether the diaper needs to be replaced.

FIG. 11 is a cross-sectional view II of a diaper according to an embodiment. Referring to FIG. 11, in an embodiment, no inner core layer 13 is provided on the coupling path between the first coil 130 and the second coil 210 in the metabolite processing apparatus. It should be appreciated that when no inner core layer 13 is provided on the coupling path, the influence of the moisture absorption status of the inner core layer 13 on the electromagnetic coupling efficiency can be avoided, thereby effectively avoiding the situation where the connection between the first coil 130 and the second coil 210 is unstable or even disconnected when the diaper is not replaced for a long time. Accordingly, in the embodiment, the reliability of communication can be effectively guaranteed by the above provided structure.

In an embodiment, the diaper may further include a receiving bag. The receiving bag is connected to an outer side of the bottom layer 12, and is configured to receive the processing module 200. The receiving bag is provided with an opening, and a switch structure is provided at the opening. The switch structure is configured to expose the opening when opened, to put in or take out the processing module 200 through the opening. The switch structure is further configured to close the opening when closed, to prevent the processing module 200 from falling off during the operation. Specifically, when the user replaces the diaper, the processing module 200 can be mounted in the receiving bag of a new diaper, thereby reducing the usage cost of the user. Moreover, compared to the wired connection mode, when the processing module 200 is removed from or mounted into the diaper of the present embodiment, there is no need to perform operations such as alignment of the connecting wires, thereby improving the convenience of removing and mounting. In addition, by upgrading a software function of the processing module 200, the diaper can support richer detection and analysis functions. It should be appreciated that after the software functions are updated based on the wireless connection mode, there is no need to improve the hardware structure such as pins and connecting wires of the processing module 200, thereby effectively improving the functional expansibility of the processing module 200 without excessively increasing the cost. In the embodiment, by providing the receiving bag, it can be ensured that the position of the processing module 200 does not move too much, thereby ensuring the reliability of the communication process. Furthermore, by providing the receiving bag with an opening having a switch structure, the processing module 200 can be prevented from naturally falling out of the receiving bag, thereby further improving the reliability.

In an embodiment, the receiving bag is provided at a position corresponding to the first coil 130 in the sensor module 100 to minimize the coupling path between the second coil 210 and the first coil 130. Through the above-mentioned arrangement mode, the transmission efficiency between the two coils can be maximized, thereby improving the communication rate in the communication process.

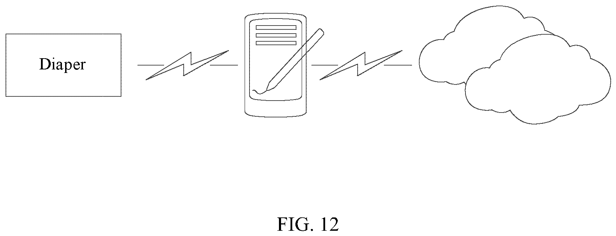

In an embodiment of the present disclosure, a nursing system is provided. FIG. 12 is a schematic structure diagram of a nursing system according to an embodiment. Referring to FIG. 12, the nursing system may include a mobile terminal and the above-mentioned diaper. The mobile terminal may include a first wireless transmission unit, and the processing module 200 of the diaper may further include a second wireless transmission unit 260 (referring to FIG. 9) which is connected to the data processing unit 220 and is configured to transmit the metabolite information to the first wireless transmission unit of the mobile terminal, and receive a control instruction from the first wireless transmission unit. The processing module 200 in the diaper serves as a host and transmits monitoring data to the APP on the mobile terminal through the wireless transmission unit. The APP on the mobile terminal can acquire the data transmitted by the processing module 200, and display the processed data on the UI interface of the APP to implement functions such as user review and feedback, etc.

Referring to FIG. 12, in an embodiment, the nursing system may further include a cloud server. The mobile terminal is further configured to transmit the metabolite information to the cloud server, and the cloud server is configured to store and analyze the received metabolite information. The APP on the mobile terminal may further forward the acquired data to the cloud, thereby implementing the cloud storage of the data and richer analysis and application decision-making functions, so that the flexibility of the use process is improved.

In the description of the specification, reference to terms "some embodiments", "other embodiments", "ideal embodiments", etc., means that the specific features, structures, materials or characteristics described in conjunction with the embodiment or example are included in at least one embodiment or example of the present disclosure. In the specification, the illustrative descriptions of the above terms do not necessarily refer to the same embodiment or example.

The technical limitations in the above-described embodiments may be arbitrarily combined. To make the description concise, all possible combinations of the technical limitations in the above-described embodiments are not described. However, as long as there is no contradiction in combinations of these technical limitations, these combinations should be considered to be within the scope of the present disclosure.

The above-described embodiments merely express several implementation modes of the present disclosure, and the description thereof is relatively specific and detailed, but it should not be construed as limiting the scope of the present disclosure. It should be pointed out that, those skilled in the art can make several modifications and improvements without departing from the concept of the present disclosure, which all fall within the scope of protection of the present disclosure. Therefore, the protection scope of the present disclosure shall be subject to the appended claims.

## Claims

1. A metabolite detection apparatus, applied to a diaper, the metabolite detection apparatus comprising:
a sensor module, comprising a sensor unit, a signal conditioner chip, and a first coil connected to each other in sequence, wherein the sensor unit is configured to sense a metabolite and generate a plurality of corresponding sensing signals, the signal conditioner chip is configured to process each of the sensing signals to generate a plurality of corresponding conditioning signals respectively, and transmit the plurality of conditioning signals to the first coil for transmissions;
a processing module, comprising a second coil and a data processing unit connected to each other, wherein the second coil is electromagnetically coupled to the first coil to wirelessly transmit energy and data, the second coil is configured to receive the plurality of conditioning signals and transmit the plurality of conditioning signals to the data processing unit, and the data processing unit is configured to acquire metabolite information according to the plurality of conditioning signals.

2. The metabolite detection apparatus according to claim 1, wherein
the sensor unit comprises a plurality of sensors, each of which is provided on a diffusion path of the metabolite, and each of the sensors is configured to generate a corresponding sensing signal;
the signal conditioner chip is configured with a plurality of sensing channel interfaces, each of which is configured to connect to a corresponding sensor.

3. The metabolite detection apparatus according to claim 2, wherein the signal conditioner chip is configured with an updateable signal conditioning algorithm, and the signal conditioning algorithm is set to correspond to types of the plurality of sensors in the sensor unit.

4. The metabolite detection apparatus according to claim 2, wherein the signal conditioner chip is provided with preset identity information,
wherein when the signal conditioner chip transmits the plurality of conditioning signals to the first coil for transmissions, the signal conditioner chip is further configured to transmit the identity information to the first coil for the transmission, to allow the processing unit to establish a mapping relationship between the metabolite information and the identity information.

5. The metabolite detection apparatus according to claim 2, wherein the data processing unit is further configured to generate and transmit a data acquisition instruction via the second coil,
the signal conditioner chip is further configured to receive the data acquisition instruction via the first coil, and transmit the conditioning signal back to the data processing unit in response to the data acquisition instruction.

6. The metabolite detection apparatus according to claim 2, wherein the sensor unit comprises:
a plurality of humidity sensors, respectively provided at different sensing points on the diffusion path of the metabolite, and each humidity sensor being configured to sense a humidity at a sensing point and generate a corresponding humidity sensing signal;
wherein the data processing unit is further configured to determine a metabolite type according to the humidity sensed by each humidity sensor, and the metabolite type comprises a liquid metabolite and a non-liquid metabolite.

7. The metabolite detection apparatus according to claim 2, wherein the sensor unit comprises:
at least one temperature sensor, provided on the diffusion path of the metabolite, wherein the temperature sensor is configured to sense a temperature at a sensing point and generate a corresponding temperature sensing signal;
wherein the data processing unit is further configured to determine a metabolite type according to the temperature sensed by the temperature sensor, the metabolite type comprises a liquid metabolite and a non-liquid metabolite.

8. The metabolite detection apparatus according to claim 2, wherein the sensor unit comprises:
a liquid-phase metabolite sensor, provided in a biochemical reaction region of the diaper, and configured to sense a liquid flowing into the biochemical reaction region from a liquid siphon channel of the diaper and generate a corresponding liquid-phase biochemical sensing signal;
wherein the data processing unit is further configured to acquire liquid-phase biochemical information corresponding to the liquid-phase biochemical sensing signal.

9. The metabolite detection apparatus according to claim 2, wherein the sensor unit comprises a gas-phase metabolite sensor and a gas latching structure covering the gas-phase metabolite sensor,
wherein the gas latching structure is configured to latch the gas in the metabolite, and the gas-phase metabolite sensor is configured to sense the latched gas and generate a corresponding gas-phase biochemical sensing signal
wherein the data processing unit is further configured to acquire gas-phase biochemical information corresponding to the gas-phase biochemical sensing signal.

10. The metabolite detection apparatus according to claim 1, wherein the processing module further comprises at least one of an operation unit, an indicator unit, or a power management unit, wherein
the operation unit is connected to the data processing unit, and is configured to receive an activation operation input by a user and generate an activation signal accordingly, and transmit the activation signal to the data processing unit;
the indicator unit is connected to the data processing unit, and is configured to receive an indication signal output by the data processing unit, and indicate the user according to status information carried by the indication signal, wherein the status information carried by the indication signal comprises at least one of status information of the metabolite, status information of the sensor module, or status information of the processing unit; and
the power management unit is connected to the data processing unit and is configured to supply power to the data processing unit.

11. A diaper, comprising:
a surface layer;
a bottom layer;
at least one inner core layer provided between the surface layer and the bottom layer;
a sensor layer, provided between the surface layer and the bottom layer, wherein at least one inner core layer is provided between the sensor layer and the bottom layer; and
the metabolite detection apparatus of any one of claims 1 to 10, wherein the sensor module in the metabolite detection apparatus is provided in the sensor layer, and the processing module in the metabolite detection apparatus is provided on an outer side of the bottom layer.

12. The diaper according to claim 11, wherein there is no inner core layer provided on a coupling path between the first coil and the second coil in the metabolite processing apparatus.

13. The diaper according to claim 11, wherein a partial inner core layer is provided on a coupling path between the first coil and the second coil in the metabolite processing apparatus;
wherein the processing unit is further configured to acquire an electromagnetic coupling efficiency on the coupling path, and acquire the amount of metabolite absorbed by the inner core layer according to the electromagnetic coupling efficiency.

14. The diaper according to claim 11, further comprising:
a receiving bag connected to an outer side of the bottom layer and configured to receive the processing module, wherein the receiving bag is provided with an opening, a switch structure is provided at the opening, the switch structure is configured to expose the opening when opened to put in or take out the processing module through the opening, and the switch structure is further configured to close the opening when closed to prevent the processing module from falling off during an operation.

15. The diaper according to claim 14, wherein the receiving bag is provided at a position corresponding to the first coil in the sensor module to minimize a coupling path between the second coil and the first coil.

16. A nursing system, comprising:
a mobile terminal comprising a first wireless transmission unit;
the diaper of any one of claims 11 to 15, wherein the processing module of the diaper further comprises a second wireless transmission unit which is connected to a data processing unit and is configured to transmit metabolite information to the first wireless transmission unit of the mobile terminal, and receive a control instruction from the first wireless transmission unit.

17. The nursing system according to claim 16, further comprising a cloud server, wherein the mobile terminal is further configured to transmit the metabolite information to the cloud server, and the cloud server is configured to store and analyze the received metabolite information.
